(19) Europäisches Patentamt
European Patent Office
Office européen des brevets

(11) Veröffentlichungsnummer: **0 144 669**
**B1**

(12) # EUROPÄISCHE PATENTSCHRIFT

(45) Veröffentlichungstag der Patentschrift: **18.03.87**

(51) Int. Cl.[4]: **C 07 D 253/06**

(21) Anmeldenummer: **84112711.1**

(22) Anmeldetag: **22.10.84**

(54) **Verfahren zur Herstellung von praktisch isomerenfreiem 4-Amino-3-ethylthio-6-tert.-butyl-1,2,4-triazin-5-on.**

(30) Priorität: **04.11.83 DE 3339858**

(43) Veröffentlichungstag der Anmeldung: **19.06.85 Patentblatt 85/25**

(45) Bekanntmachung des Hinweises auf die Patenterteilung: **18.03.87 Patentblatt 87/12**

(84) Benannte Vertragsstaaten: **BE CH DE FR GB IT LI NL**

(56) Entgegenhaltungen:
**EP-A-0 074 538**
**US-A-3 897 429**
**US-A-3 905 973**
**US-A-3 966 715**
**US-A-4 058 525**
**US-A-4 175 188**
**US-A-4 309 538**
**US-A-4 328 340**
**US-B-514 259**

(73) Patentinhaber: **BAYER AG, Konzernverwaltung RP Patentabteilung, D-5090 Leverkusen 1 Bayerwerk (DE)**

(72) Erfinder: **Schmidt, Thomas, Dr., Ginsterweg 9, D-5657 Haan 1 (DE)**

EP 0 144 669 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein auch technisch einsetzbares Verfahren zur Herstellung von praktisch isomerenfreiem 4-Amino-3-ethylthio-6-tert.-butyl-1,2,4-triazin-5-on (I), einem bekannten herbiziden Wirkstoff, der nach der neuen Methode sehr selektiv aus dem Reaktionsgemisch isoliert werden kann, ohne nennenswerte Verunreinigung durch das unerwünschte, nicht herbizid wirksame, N-ethylierte 2-Ethyl-3-thioxo-Derivat (Ia).

Es ist bereits bekannt, daß man das 4-Amino-3-ethyl-thio-6-tert.-butyl-1,2,4-triazin-5-on (I) erhält, wenn man 4-Amino-3-mercapto-6-tert.-butyl-1,2,4-triazin-5-on (II) in alkalischer Lösung mit einem Ethylierungsmittel, wie Ethyliodid oder -bromid, umsetzt (vgl. z.B. US-PS 3 671 523). Dieses Verfahren hat jedoch den Nachteil, daß neben dem gewünschten S-ethylierten Endprodukt der Formel (I) ca. 15 % an unerwünschtem N-ethyliertem Nebenprodukt der Formel (Ia) entstehen:

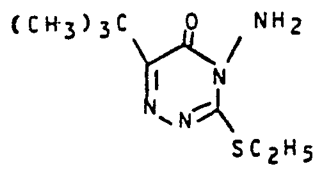

(I)

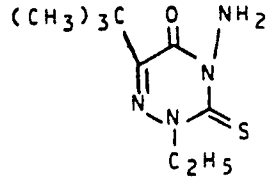

(Ia)

Ein weiterer Nachteil besteht darin, daß dieses Gemisch mit herkömmlichen technischen Methoden nur schlecht trennbar ist. Durch ein verlustreiches, aufwendiges Umkristallisieren ist in einem Schritt lediglich eine Herabsetzung an N-ethyliertem Nebenprodukt der Formel (Ia) auf ca. 6 % möglich.

Es wurde nun gefunden, daß man das bekannte 4-Amino-3-ethylthio-6-tert.-butyl-1,2,4-triazin-5-on der Formel (I)

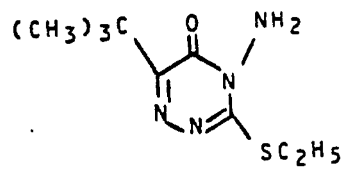

(I)

durch Umsetzung von 4-Amino-3-mercapto-6-tert.-butyl-1,2,4-triazin-5-on der Formel (II) - das in zwei tautomeren Formen auftreten kann-

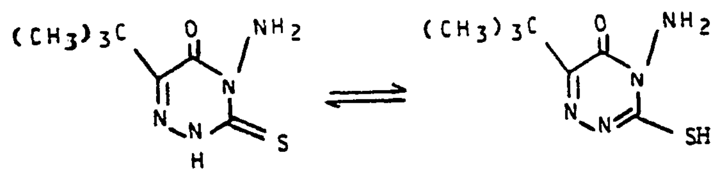

(II)

mit einem Ethylierungsmittel in wäßrig-alkalischer Lösung, auch in technischem Maßstab, in guter Ausbeute und Reinheit dann erhält, wenn man das gut gerührte Reaktionsgemisch bei einer Temperatur von 40 - 60° C mit wenigstens 50 g Waschbenzin - bezogen auf 1 Mol Ausgangsverbindung (II) - versetzt, das nunmehr aus zwei flüssigen Phasen bestehende Reaktionsgemisch anschließend auf Raumtemperatur abkühlt und das hierbei auskristallisierende reine Endprodukt der Formel (I) in üblicher Weise isoliert.

Es ist als ausgesprochen überraschend zu bezeichnen, daß sich das 4-Amino-3-ethylthio-6-tert.-butyl-1,2,4-triazin-5-on der Formel (I) durch dieses Verfahren in guter Ausbeute und Reinheit erhalten läßt, da - wie eigene Versuche gezeigt haben - die Umkristallisation des isolierten und getrockneten Rohwirkstoffes (bestehend aus den Produkten (I) und (Ia) etwa im Verhältnis 85:15) aus Waschbenzin nur einen unbefriedigenden Reinigungseffekt bringt. Überraschenderweise kann Waschbenzin - wie weitere Versuche gezeigt haben - nicht durch andere ähnliche Lösungsmittel wie z.B. Cyclohexan ersetzt werden, ohne daß man wesentlich schlechtere Reinigungseffekte erzielt.

Weiterhin ist es überraschend, daß die gleiche Methode im Falle der dem Isomerenpaar (I)/(Ia) entsprechenden S- und N-Methylverbindungen nicht zu einer erfolgreichen Isomerentrennung angewandt werden kann. Dies gilt z.B. für den technischen Wirkstoff 4-Amino-3-methylthio-6-tert.-butyl-1,2,4-triazin-5-on, der aus der technischen Synthese ca. 6 % isomeres 4-Amino-2-methyl-3-thioxo-6-tert.-butyl-1,2,4-triazin-5-on enthält.

Das erfindungsgemäße Verfahren hat den Vorteil eines hohen Reinigungseffektes bei sehr geringem Produktverlust. Das gewünschte Produkt wird - auch bei technischer Herstellung - mit einem Gehalt von ca. 97 % erhalten, bei gleichzeitiger guter Raum/Zeit-Ausbeute. Ein weiterer Vorteil ist die Einfachheit der Verfahrensdurchführung, wodurch ein separates, aufwendiges Umkristallisieren vermieden werden kann.

Das als Ausgangsstoff zu verwendende 4-Amino-3-mercapto-6-tert.-butyl-1,2,4-triazin-5-on der Formel (II) ist bekannt (vgl. z.B. US-PS 3 671 523). Als Ethylierungsmittel kommen vorzugsweise infrage: Ethylhalogenide, wie Ethylchlorid, -bromid und -iodid; ferner Diethylsulfat, Triethylphosphat, Triethylphosphit, Methansulfonsäureethylester und p-Toluolsulfonsäureethylester.

Die Umsetzung mit dem Ethylierungsmittel wird in Gegenwart einer Base durchgeführt. Hierbei werden bevorzugt alle üblichen starken anorganischen Basen eingesetzt, wie Natriumhydroxid, Kaliumhydroxid oder Calciumhydroxid, jeweils in wässriger Lösung.

Bei der Durchführung der Ethylierung setzt man auf 1 Mol der Verbindung der Formel (II) 0,1 bis 5, vorzugsweise 0,3 bis 2 Mol Ethylierungsmittel und 1 bis 10 Mol, vorzugsweise 1 bis 5 Mol wässrige Base ein.

Das Reaktionsgemisch wird vor, während oder nach der Ethylierung bei einer Temperatur von 40 - 60°C, vorzugsweise von 40 - 50°C, unter kräftigem Rühren mit Waschbenzin versetzt. Als Waschbenzin können verschiedene Fraktionen der technischen Kohlenwasserstoffdestillation eingesetzt werden, wie insbesondere die Fraktionen im Siedebereich zwischen 100 und 140°C. Man setzt auf 1 Mol Ausgangsverbindung der Formel (II) im allgemeinen 50 - 400 g, bevorzugt 100 - 250 g Waschbenzin ein. Das erfindungsgemäße Verfahren kann besonders zweckmäßig wie folgt durchgeführt werden:

1 Mol 4-Amino-3-mercapto-6-tert.-butyl-1,2,4-triazin-5-on der Formel (II) wird in der angegebenen Menge wässriger Base gelöst und mit 0,3 bis 2 Mol Ethylierungsmittel versetzt. Die Reaktionszeit beträgt zwei bis acht Stunden bei einer Temperatur von 40 - 60°C. Das Reaktionsgemisch wird mit der angegebenen Menge Waschbenzin versetzt und auf ca. 20°C abgekühlt, wobei das gewünschte Endprodukt der Formel (I) auskristallisiert. Der Feststoff wird abfiltriert, neutral gewaschen und im Vakuum getrocknet.

Hervorzuheben ist, daß die Reaktion nicht bis zur vollständigen Umsetzung des Ausgangsstoffes der Formel (II) durchgeführt werden muß, sondern daß sie auch vorzeitig abgebrochen werden kann. Nicht umgesetzter Ausgangsstoff der Formel (II) bleibt in Lösung und kann nach Abtrennung der Waschbenzinphase mit der Mutterlauge im nächsten Ansatz wieder eingesetzt werden.

Die Verbindung der Formel (I) weist bekanntermaßen eine sehr gute herbizide Wirksamkeit auf (vgl. US-PS 3 671 523).

Das erfindungsgemäße Verfahren - im Vergleich zum Stand der Technik - sei anhand des folgenden Herstellungsbeispiels näher erläutert:

**Herstellungsbeispiel**

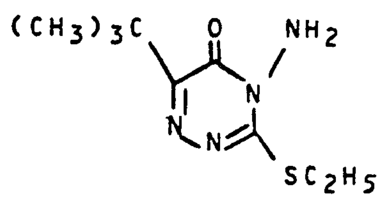

(I)

**a) erfindungsgemäß** (in technischem Maßstab):

In einem 1250 L-Rührwerkskessel werden 147,15 kg (0,736 kMol) 4-Amino-3-mercapto-6-tert.-butyl-1,2,4-triazin-5-on (II), 600 kg Wasser, 75 kg 45 %ige Natronlauge und 90 kg (0,826 kMol) Ethylbromid 5 Stunden bei einer Innentemperatur von 40 - 50°C gerührt. Anschließend gibt man bei dieser Temperatur 167 kg Waschbenzin (Fraktion der tech. Kohlenwasserstoffdestillation im Siedebereich zwischen 100 und 140°C) in den Kessel und kühlt auf ca. 20°C ab. Der auskristallisierte Feststoff wird abfiltriert, dreimal mit je 150 kg 1 %iger Natronlauge und dreimal mit je 150 kg Wasser gewaschen und im Vakuum getrocknet.

Man erhält 113,2 kg (78 % der Theorie, bezogen auf umgesetztes Ausgangsprodukt der Formel (II) 4-Amino-3-ethylthio-6-tert.-butyl-1,2,4-triazin-5-on vom Schmelzpunkt 92 - 94°C mit einer Zusammensetzung (gaschromatographisch bestimmt) von 96,9 % 4-Amino-3-ethylthio-6-tert.-butyl-1,2,4-triazin-5-on (I) und 2,7 % 4-Amino-2-ethyl-3-thioxo-6-tert.-butyl-1,2,4-triazin-5-on (Ia).

Aus den vereinigten Mutterlaugen und Waschwässern können durch Ansäuren in üblicher Weise 20 kg 4-Amino-3-mercapto-6-tert.-butyl-1,2,4-triazin-5-on (II) zurückgewonnen werden.

Die Waschbenzinphase enthält 19,1 kg 4-Amino-2-ethyl-3-thioxo-6-tert.-butyl-1,2,4-triazin-5-on (Ia) (13,2 % der Theorie, bezogen auf umgesetztes Ausgangsprodukt der Formel (II)) und 0,83 kg 4-Amino-3-ethyl-thio-6-tert.-butyl-1,2,4-triazin-5-on (I) (0,57 % der Theorie, bezogen auf umgesetztes Ausgangsprodukt der Formel (II)).

**b) nach dem Stand der Technik** (Vergleichsversuch):

In einem 2 L-Dreihalskolben werden 200 g (1 Mol) 4-Amino-3-mercapto-6-tert.-butyl-1,2,4-triazin-5-on (II) in 1800 ml Wasser und 60 g 25 %iger Natronlauge gelöst und bei 45°C mit 128 g (1,17 Mol) Ethylbromid versetzt. Nach Zugabe weiterer 114 g 25 %iger Natronlauge läßt man das Reaktionsgemisch 8 Stunden bei 45°C nachrühren, kühlt auf 20°C ab und filtriert. Der zurückbleibende Feststoff wird zweimal mit je 180 ml 1 %iger Natronlauge und dreimal mit je 180 ml Wasser gewaschen und im Vakuum bei 50°C getrocknet. Man erhält 182 g (75,7 % der Theorie, bezogen auf umgesetztes Ausgangsprodukt der Formel (II)) 4-Amino-3-ethylthio-6-tert.-butyl-1,2,4-triazin-5-on mit einer Zusammensetzung (gaschromatographisch bestimmt) von 85,54 % 4-Amino-3-ethylthio-6-tert.-butyl-1,2,4-triazin-5-on (I) und 14,46 % 4-Amino-2-ethyl-3-thioxo-6-tert.-butyl-1,2,4-triazin-5-on (Ia).

Aus der Mutterlauge können durch Ansäuern und übliche Aufarbeitung 19,6 g 4-Amino-3-mercapto-6-tert.-butyl-1,2,4-triazin-5-on (II) zurückgewonnen werden.

## Patentansprüche

1) Verfahren zur Herstellung von praktisch isomerenfreiem 4-Amino-3-ethylthio-6-tert.-butyl-1,2,4-triazin-5-on (I) durch Umsetzung von 4-Amino-3-mercapto-6-tert.-butyl-1,2,4-triazin-5-on (II) mit einem Ethylierungsmittel in wäßrig-alkalischer Lösung, dadurch gekennzeichnet, daß man das gut gerührte Reaktionsgemisch bei einer Temperatur von 40 - 60 °C mit wenigstens 50 g Waschbenzin - bezogen auf 1 Mol Ausgangsverbindung (II) - versetzt, das nunmehr aus zwei flüssigen Phasen bestehende Reaktionsgemisch anschließend auf Raumtemperatur abkühlt und das hierbei auskristallisierende reine Endprodukt der Formel (I) in üblicher Weise isoliert.

2) Verfahren nach Anspruch 1, dadurch gekennzeichnet, daß man bei einer Temperatur von 40 - 50 °C arbeitet.

3) Verfahren nach Anspruch 1, dadurch gekennzeichnet, daß man pro Mol Ausgangsverbindung (II) 50 - 400 g Waschbenzin einsetzt.

4) Verfahren nach Anspruch 3, dadurch gekennzeichnet, daß man pro Mol Ausgangsverbindung (II) 100 - 250 g Waschbenzin einsetzt.

5) Verfahren nach Anspruch 1, dadurch gekennzeichnet, daß man Waschbenzin mit einem Siedebereich von 100 - 140°C verwendet.

## Claims

1) Process for preparing virtually isomer-free 4-amino-3-ethylthio-6-tert.-butyl-1,2,4-triazin-5-one (I) by reacting 4-amino-3-mercapto-6-tert.-butyl-1,2,4-triazin-5-one- (II) with an ethylating agent in aqueuus alkali solution, characterised in that at least 50 g of petroleum ether - relative to 1 mole of starting compound (II) - are added at a temperature of 40 - 60°C to the thoroughly stirred reaction mixture, the reaction mixture, now consisting of two liquid phases, is then cooled down to room temperature, and the pure end product of the formula (I) which crystallises out in the course of the cooling is isolated in conventional manner.

2) Process according to Claim 1, characterised in that the reaction is carried out at a temperature of 40-50°C.

3) Process according to Claim 1, characterised in that 50 - 400 g of petroleum ether are used per mole of starting compound (II).

4) Process according to Claim 3, characterised in that 100 - 250 g of petroleum ether are used per mole of starting compound (II).

5) Process according to Claim 1, characterised in that the petroleum ether used has a boiling range from 100 to 140°C.

## Revendications

1. Procédé de fabrication de 4-amino-3-éthylthio-6-t-butyl-1,2,4-triazine-5-one(I) pratiquement exempte d'isomères, par réaction de la 4-amino-3-mercapto-6-t-butyl-1,2,4-triazine-5-one (II) avec un agent d'éthylation en solution alcaline aqueuse, caractérisé en ce qu'au mélange de réaction bien agité on ajoute à une température de 40 - 60°C au moins 50 g d'essence rectifiée - par mole du composé de départ (II) - on refroidit ensuite le mélange de réaction à la température ordinaire, qui se compose désormais, de deux phases liquides, et l'on isole de la manière en usage le produit final pur de formule (I) qui s'est séparé par cristallisation.

2. Procédé selon la revendication 1, caractérisé en ce qu'on opère à une température de 40 - 50°C.

3. Procédé selon la revendication 1, caractérisé en ce que par mole du composé de départ (II) on utilise 50 - 400 g d'essence rectifiée.

4. Procédé selon la revendication 3, caractérisé en ce que par mole du composé de départ (II) on utilise 100 - 250 g d'essence rectifiée.

5. Procédé selon la revendication 1, caractérisé en ce qu'on utilise une essence rectifiée ayant un intervalle d'ébullition de 100 - 140° C.